# FASCICULE DE BREVET EUROPEEN

(11) **EP 1 073 415 B1**
(45) Date de publication et mention de la délivrance du brevet: **26.10.2005**
(21) Numéro de dépôt: 99923418.0
(22) Date de dépôt: 16.04.1999
(51) Int. Cl.: A61K 9/10, A61K 47/12, A61K 47/24

(54) **COMPOSITIONS PHARMACEUTIQUES GELIFIABLES**
GELIERBARE PHARMAZEUTISCHE ZUSAMMENSETZUNGEN
PHARMACEUTICAL COMPOSITIONS CAPABLE OF BEING GELLED

(30) Priorité: 30.04.1998 BE 3200009
(43) Date de publication de la demande: 07.02.2001
(73) Titulaire: UCB, S.A., 1070 Bruxelles (BE)
(72) Inventeur: FANARA, Domenico, B-4520 Wanze (BE); VRANCKX, Henri, B-1190 Bruxelles (BE); DELEERS, Michel, B-1630 Linkebeek (BE)
(74) Mandataire: Lechien, Monique
(86) Numéro de dépôt international: PCT/EP1999/002551
(87) Numéro de publication internationale: WO 1999/056725

(56) Documents cités:
- EP-A- 0 429 224
- EP-A- 0 550 960
- WO-A-94/10978
- WO-A-95/03787
- WO-A-97/15285

## Description

La présente invention se rapporte à des compositions pharmaceutiques permettant la libération prolongée d'au moins une substance active, à des procédés de préparation de ces compositions, ainsi qu'à leur utilisation pour l'administration de médicaments par voie sous-cutanée et/ou intramusculaire.

Les deux voies extra-vasculaires principales d'administration parentérale sont les voies sous-cutanée et intramusculaire. Par rapport à l'injection intraveineuse, ces deux voies d'administration pour une même solution aqueuse de principe actif produisent généralement un effet légèrement différé et légèrement prolongé. La biodisponibilité du médicament est aussi généralement inférieure en raison d'une absorption plus lente, une fixation ou une dégradation du médicament au site d'injection ou dans les tissus traversés. Ainsi la TRH (thyrotropin releasing hormone, un tripeptide) a une biodisponibilité chez la souris de 67,5 % après administration sous-cutanée et de 31,4 % après administration intramusculaire (Redding T.W. and Schally A.V., Life Sci., 12, 23 (1970)).

Pour améliorer la biodisponibilité et obtenir de véritables préparations à libération prolongée, différentes formes expérimentales ont été développées.

Ainsi l'encapsulation par des liposomes de P-18, un peptide de poids moléculaire inférieur à 5000 Daltons, montre qu'après injection intramusculaire, le peptide demeure 7 jours au niveau du site d'injection (Crommelin D.J.A. and Storm G., Int. Pharm J., 1. 179 (1987)).

Un autre moyen de prolonger la libération d'un principe actif consiste en son incorporation dans un implant. Ces implants peuvent être préparés à partir de polymères biodégradables ou non. L'inconvénient de cette forme est lié à son mode d'introduction sous cutanée par incision ou à l'aide d'un trocart. De plus, si un polymère non biodégradable est utilisé, l'implant doit être retiré par incision après diffusion de la totalité du principe actif hors de la matrice polymérique. Ces systèmes ont été largement développés pour l'administrations d'hormones telle la LHRH (luteinizing hormone releasing hormone) et ses analogues synthétiques. Ainsi la goséréline administrée chez l'homme sous forme d'implants de PLA-GA (copolymère d'acide lactique et d'acide glycolique) permet une diminution très importante et durable du taux de testostérone dans le sang (Vogelzang N.J., Chodak G.W., Soloway M.S., Block N.L.. Schellhammer P.F., Smith J.A., Caplan R.J. and Kennealey G.T., Urology, 46, 220 (1995)).

D'autres supports polymériques peuvent encore être utilisés: les micro ou nanoparticules. Dans ce cas, seuls les polymères biodégradables sont utilisés. Par rapport aux implants, ces particules peuvent être injectées à l'aide d'une seringue classique mais présentent l'inconvénient de ne pouvoir être retirées de l'organisme en cas de problème. Une diminution très importante et durable du taux de testostérone a également été observée chez l'homme après administration de microparticules de PLA-GA contenant de la nafaréline.

Ces différents systèmes d'administration présentent l'inconvénient d'une préparation sophistiquée et complexe qui demande des installations particulières.

La demanderesse vient maintenant de découvrir de nouvelles compositions pharmaceutiques, obtenues par un procédé de préparation extrêmement simple et qui permettent une libération prolongée d'un principe actif. Ces compositions ont la propriété de gélifier instantanément en présence d'une phase aqueuse. Elles peuvent donc être judicieusement utilisées pour obtenir, par les voies sous-cutanée et intramusculaire, une libération soutenue et programmée de médicaments. Au contact des muqueuses, un gel va se former sous la peau ou dans le muscle et le médicament pourra diffuser et être libéré à partir du gel.

Des compositions lipidiques qui subissent une transformation de phase au contact de l'eau ont déjà été présentées dans la littérature.

La demande de brevet européen 550960 décrit des compositions pour application topique, destinées à éviter la transpiration, comprenant un agent évitant la transpiration, qui comprend au moins une substance amphiphile, cet agent évitant la transpiration étant capable de former une phase cristalline liquide insoluble dans l'eau, ayant une périodicité supérieure à 1. En particulier, l'exemple 14 illustre une composition capable de former une phase cristalline hexagonale inverse au contact de la transpiration, composée de 34 à 50 % d'acide oléique et de 50 à 66% de lécithine (phosphatidylcholine).

La demande de brevet internationale WO 94/10978 décrit des compositions émulsifiantes destinées à remplacer les émulsifiants synthétiques couramment utilisés dans l'industrie alimentaire, cosmétique, de toilettage ou pharmaceutique. Ces compositions comprennent au moins un lipide membranaire (phospholipide), au moins un amphiphile naturel qui ne soit pas un émulsifiant primaire (acide gras ou alcool gras en C₁₂ à C₂₂, ou combinaison d'un acide gras et d'un alcool gras), et, optionnellement, un milieu hydrophile (alcool aliphatique tel que le propylène glycol). Ces compositions possèdent la propriété de former des crèmes (émulsion huile dans l'eau) avec des huiles ou des substances huileuses et sont capables de former des émulsions stables ou des crèmes lorsqu'elles sont mélangées avec des liposomes.

Plus particulièrement, l'exemple 4 décrit une composition constituée de 15-% en poids de lécithine de soja hydrogénée (phospholipide), 15 % en poids d'acide gras, 45% en poids d'alcool gras et 25% en poids d'alcool (10% d'éthanol et 15% de glycérol). Cette composition se présente sous la forme d'une cire (soft waxy mass).

La littérature mentionne également des compositions pharmaceutiques fluides destinées au traitement des paradontites, se présentant sous la forme de suspensions ou émulsions plus ou moins visqueuses qui sont administrées dans la poche parodontale, généralement à l'aide de seringues.

La demande de brevet internationale WO 95/34287 décrit des compositions lipidiques biodégradables sous la forme de phases cristallines L2, permettant la libération contrôlée de substances actives et comprenant, outre la substance active, au moins un diacyl glycérol d'acide gras insaturé ayant de 16 à 22 atomes de carbones ou d'acide gras saturé ayant de 14 à 22 atomes de carbone, et au moins un phospholipide choisi parmi les glycérophosphatides et sphingophosphatides, et, éventuellement, au moins un liquide polaire choisi parmi l'eau, le glycérol, l'éthylène glycol et le propylène glycol. Ces compositions possèdent la particularité de se transformer au contact de l'eau en phases cristallines liquides cubiques, ce qui permet de "mouler" la substance active dans le site où l'on désire que l'action ait lieu. Ce document mentionne, parmi d'autres applications, la possibilité d'utiliser de telles compositions pour le traitement de la parodontite. Toutefois, l'efficacité de telles compositions dans le traitement de la parodontite n'est pas illustrée dans ce document.

Le brevet européen 429224 décrit des compositions se présentant sous la forme de gels contenant de 1 à 99% en poids de monooléine et de 1 à 90% en poids de substance active, que l'on place dans la cavité parodontale. En présence de l'eau environnante, ces compositions deviennent plus visqueuses et maintiennent la substance active près de son site d'action. La substance active est libérée lentement de manière contrôlée.

Le brevet US 5230895 décrit l'utilisation de compositions se présentant sous la forme de solutions ou de pâtes capables de se transformer en gel lorsqu'elles ont été placées dans la poche parodontale. Ces compositions sont biodégradables et permettent la libération contrôlée de la substance active, dans le site d'action. Elles contiennent un mélange de glycérides et d'une substance active choisi de manière telle qu'il soit capable de former un gel dans l'environnement de la poche parodontale. Les compositions illustrées dans ce document contiennent au moins 70% de Myverol™ 18-92 qui est une composition de monoglycérides de tournesol ayant une teneur en monoglycéride d'au moins 90%.

Le brevet US 5143934 décrit des compositions permettant l'administration par libération contrôlée d'une substance active dans une poche parodontale, comprenant au moins un monoglycéride et au moins une huile végétale dans des proportions suffisantes pour former une phase cristalline liquide au contact de l'eau présente dans la poche parodontale. Ces compositions sont solides à température ambiante, mais elles ont un point de fusion inférieur à la température corporelle.

La présente invention concerne des compositions pharmaceutiques fluides permettant la libération contrôlée d'au moins une substance active comprenant
a) une quantité thérapeutiquement efficace d'au moins une substance active,
b) de 3 à 55% en poids de phospholipide,
c) de 16 à 72% en poids de solvant pharmaceutiquement acceptable, et
d) de 4 à 52% en poids d'acide gras,
ces compositions ayant la propriété de se gélifier instantanément en présence d'une phase aqueuse.

Selon un autre aspect, l'invention se rapporte à des procédés pour la préparation de ces compositions.

Selon un troisième aspect, l'invention se rapporte à l'utilisation de ces compositions pour la libération contrôlée d'une ou plusieurs substances actives par injection sous-cutanée et/ou intramusculaire.

Les compositions selon la présente invention comprennent une quantité thérapeutiquement efficace d'au moins une substance active. Cette dernière peut être liposoluble ou hydrosoluble. On citera à titre d'exemple des antibiotiques, en particulier les antibiotiques actifs contre les bactéries anaérobies tels que la doxycycline ou la minocycline et leurs sels pharmaceutiquement acceptables, des agents anti-infectieux, tels que le métronidazole, la chlorhexidine, le chlorure de benzalkonium, le p-chloro-m-crésol, l'alcool 1,2-dichlorobenzylique, l'hexamidine ou le chloroféne et leurs sels pharmaceutiquement acceptables, des anesthésiques locaux tels que la lidocaïne, la procaïne, la tétracaine, l'articaïne, la bupivacaïne, la mépivacaïne ou la prilocaïne et leurs sels pharmaceutiquement acceptables, des anti-inflammatoires stéroïdiens ou autres, tels que l'hydrocortisone, la cortisone, la prednisone, la prednisolone, la méthylprednisolone, la triamcinolone, la bétaméthasone ou la dexaméthasone et leurs sels pharmaceutiquement acceptables ainsi que l'acéclofénac, le diclofénac, l'ibuprofène et le piroxicam et leurs sels pharmaceutiquement acceptables, des antimycosiques tels que la griséofulvine, l'amphotéricine B, la natamycine, la nystatine et leurs sels pharmaceutiquement acceptables ou encore des substances actives peptidiques telles que la calcitonine, la somatostatine, l'insuline, la bone growth hormone et autres facteurs de croissance ou de réparation.

Les compositions selon la présente invention contiennent de 3 à 55% de phospholipide. Les phospholipides utilisables selon la présente invention sont des esters phosphoriques de polyols et d'acides gras. Ils peuvent provenir de sources très variées, tant naturelles que par voie de synthèse. Les phospholipides peuvent être hydrogénés ou non hydrogénés. On citera à titre d'exemples, la phosphatidylcholine, la phosphatidylcholine hydrogénée, les sels du phosphatidylglycérol, la dicaproylphosphatidylcholine ou les sels du distéaroylphosphatidylglycérol. On peut également utiliser ces phospholipides en mélange. De préférence, le phospholipide présent dans les compositions selon la présente invention est la phosphatidylcholine.

Lorsque le phospholipide est choisi parmi la phosphatidylcholine, les sels du phosphatidylglycérol, la dicaproylphosphatidylcholine ou les sels du distéaroylphosphatidylglycérol, les compositions préférées selon la présente invention contiennent de 15 à 55% en poids de phospholipide. Lorsque le phospholipide est une phosphatidylcholine hydrogénée, les compositions selon la présente invention contiennent de 3 à 11%, de préférence de 3 à 10% en poids de phospholipide.

Les compositions selon la présente invention contiennent un ou plusieurs solvants pharmaceutiquement acceptables. Par solvant pharmaceutiquement acceptable, on entend un solvant tel que le propylène glycol, les polyéthylène glycols, les huiles minérales telles que l'huile de paraffine ou les huiles de silicone ou tout autre solvant dans lequel le phospholide utilisé est soluble. On peut également utiliser des mélanges de plusieurs solvants pharmaceutiquement acceptables. On utilise de préférence le propylène glycol. Le solvant utilisé est pharmaceutiquement acceptable, ce qui signifie que le solvant ne produira pas de réaction biologique se traduisant par des infections, inflammations ou autres phénomènes de rejet.

Les compositions selon la présente invention contiennent également de 4 à 52% d'au moins un acide gras. Les acides gras utilisables selon la présente invention sont des acides organiques carboxyliques saturés ou insaturés contenant de 4 à 22 atomes de carbone, de préférence de 8 à 18 atomes de carbone. A titre d'exemple, on citera l'acide oléique, l'acide caprylique, l'acide caprique, l'acide caproïque, l'acide myristique, l'acide butyrique... On peut également utiliser des mélanges d'acides gras. L'acide gras préféré selon la présente invention est l'acide oléique.

Eventuellement, les compositions selon la présente invention peuvent aussi contenir jusqu'à 15% en poids d'eau. On notera que la quantité d'eau présente dans les compositions selon l'invention est choisie de manière à ce que la composition ait la consistance désirée pour l'application envisagée.

La demanderesse a également découvert que des phospholipides se présentant sous la forme de mélanges commerciaux conviennent pour les compositions selon la présente invention. Comme exemple de telles compositions commerciales, on citera Phosal 50 PG™ (55,8% de phosphatidylcholine. 1,9% d'acides gras de soja. 2,9% de monoglycérides de tournesol. 1,9% d'éthanol. 37.3% de propylène glycol. 0,2% de palmitate d'ascorbyle). Phosal 53 MCT™ (60.8% de phosphatidylcholine. 2% acide oléique, 3% de monoglycérides de tournesol. 5% d'éthanol, 29% de triglycérides, 0,2% de palmitate d'ascorbyle), disponibles chez NATTERMANN PHOSPHOLIPID GmbH.

Les compositions selon la présente invention peuvent également contenir les composants optionnels suivants: jusqu'à 5% en poids de monoglycéride ou de diglycéride ou d'un mélange de mono- et de diglycéride et/ou jusqu'à 15% en poids de triglycérides.

Les compositions selon la présente invention peuvent en outre contenir un ou plusieurs agents conservateurs (tel que l'éthanol), un ou plusieurs agents antioxydants (tel que le palmitate d'ascorbyle) ou un ou plusieurs agents complexants (tel que l'EDTA (éthylènediamine tétraacétate)).

Les compositions selon la présente invention permettent la libération contrôlée d'au moins une substance active. Par libération contrôlée, on entend un profil de libération de la substance active souhaitable pour le traitement envisagé. La libération de la substance active peut donc être plus ou moins retenue ou ralentie en fonction de la substance active utilisée et de l'effet thérapeutique recherché. On notera que le contrôle de la libération de la substance active peut être aisément obtenu par de simples variations des proportions des composants des compositions selon la présente invention. Elles se prêtent donc très bien à diverses applications thérapeutiques dans lesquelles on recherche la libération contrôlée d'une substance active dans un site biologique bien précis.

Les compositions selon la présente invention sont des compositions pharmaceutiques fluides se présentant sous la forme d'émulsions, de suspensions ou de préparations huileuses. Elles possèdent la propriété de se gélifier instantanément en présence d'une phase aqueuse. En effet, lorsque les compositions selon la présente invention sont placées en présence d'un excès de phase aqueuse, elles passent d'un état fluide à un état de gel non miscible avec la phase aqueuse environnante.

Selon un autre aspect, la présente invention concerne des procédés de préparation des compositions selon la présente invention. Les compositions selon la présente invention sont obtenues par un procédé comportant les étapes successives suivantes:
i) le ou les phospholipides sont dissous dans le ou les solvants pharmaceutiquement acceptables;
ii) le ou les acides gras sont ajoutés à la solution de phospholipide sous agitation;
iii) la ou les substances actives sont incorporées au mélange obtenu à la fin de l'étape ii), et
iv) de l'eau est éventuellement ajoutée à la composition obtenue à l'étape iii).

Lorsque la substance active est hydrosoluble, elle est dissoute dans une quantité minimale d'eau avant l'incorporation à l'étape iii). Lorsque la substance active n'est pas soluble dans l'eau, elle est incorporée à l'étape iii) dans le mélange de phospholipide, de solvant pharmaceutiquement acceptable et d'acide gras. Dans le cas d'une substance à la fois non hydrosoluble et peu ou pas liposoluble, elle est également incorporée à l'étape iii), éventuellement sous forme micronisée.

Les exemples qui suivent illustrent la présente invention sans toutefois la limiter. Dans ces exemples, toutes les parties sont exprimées en poids. Les produits commerciaux suivants ont été obtenus chez NATTERMANN PHOSPHOLIPID GmbH et possèdent les compositions suivantes (pourcentages en poids):
- Phospholipon 90™ : phosphatidylcholine;
- Phosal 50 PG™ : 55,8% de phosphatidylcholine, 1,9% d'acides gras de soja, 2,9% de monoglycérides de tournesol, 1,9% d'éthanol, 37,3% de propylène glycol, 0,2% de palimitate d'ascorbyle;
- NAT 8449™ : 60% de phosphatidylcholine, 40% de propylène glycol ;
- Phosal 53 MCT™ : 60,8% de phosphatidylcholine, 2% d'acide oléique, 3% de monoglycérides de tournesol. 5% d'éthanol, 29% de triglycérides, 0,2% de palmitate d'ascorbyle;
- Phospholipon G-Na™ : sel sodique de 3(3-sn-phosphatidyl)glycérol de soja;
- Phospholipon CC™ : 1.2-dicaproyl-sn-glycéro(3)phosphocholine;
- Phospholipon SG-Na™ : sel sodique de 1.2-distéaroyl-sn-glycéro(3)phospholglycérol;
- Phospholipon 90 H™ : (3-sn-phosphatidyl)choline de soja hydrogénée.

### Exemple 1.

Cet exemple illustre la préparation de diverses compositions selon l'invention. Les compositions décrites ci-après se présentent sous la forme d'émulsions, suspensions ou solutions plus ou moins visqueuses qui gélifient instantanément en présence d'une phase aqueuse.

### Mode opératoire général a:

Le Phosal 50 PG™ ou le NAT 8449™ et l'acide oléique sont mélangés sous agitation. La substance active est introduite dans le mélange sous agitation. Après homogénéisation, on ajoute éventuellement de l'eau pour rendre la préparation plus visqueuse.

### Mode opératoire général b:

Le Phosal 50 PG™ ou le NAT 8449™ et l'acide oléique sont mélangés sous agitation. La substance active est dissoute dans l'eau, et la solution ainsi obtenue est introduite dans le mélange Phosal 50 PG™ ou NAT 8449™ - acide oléique sous agitation.

### 1.1. Préparation au benzoate de métronidazole.

Les préparations ayant les compositions présentées dans le Tableau 1 sont obtenues suivant le mode opératoire général a.

**Tableau 1 -**

| Compositions A et B - Métronidazole (parties) | | | | | |
|---|---|---|---|---|---|
| Composition | A₁ | A₂ | A₃ | B₁ | B₂ |
| Phosal 50 PG™ | 54,6 | 77,4 | 81,9 | - | - |
| NAT 8449™ | - | - | - | 72,8 | 45,5 |
| acide oléique | 36,4 | 13,6 | 9,1 | 18,2 | 45,5 |
| benzoate de metrodinazole | 5,0 | 5,0 | 5,0 | 5,0 | 5,0 |
| eau | 4,0 | 4,0 | 4,0 | 4,0 | 4,0 |

### 1.2 Préparation au diacétate de chlorhexidine.

Les préparations ayant les compositions présentées dans le Tableau 2 sont obtenues suivant le mode opératoire général a.

**Tableau 2 -**

| Compositions C et D - chlorhexidine (parties) | | | | |
|---|---|---|---|---|
| Composition | C₁ | C₂ | D₁ | D₂ |
| Phosal 50 PG™ | 51,0 | 63,8 | - | - |
| NAT 8449™ | - | - | 59.5 | 51.0 |
| acide oléique | 34,0 | 21.2 | 25,5 | 34,0 |
| diacétate de chlorhexidine | 15,0 | 15,0 | 15,0 | 15,0 |

### 1.3. Préparation à l'hyclate de doxycycline.

Les préparations ayant les compositions présentées dans le Tableau 3 sont obtenues suivant le mode opératoire général b.

**Tableau 3 -**

| Compositions E et F - Doxycycline (parties) | | | | |
|---|---|---|---|---|
| Composition | E₁ | E₂ | F₁ | F₂ |
| Phosal 50 PG™ | 43,0 | 64,5 | - | - |
| NAT 8449™ | - | - | 51,6 | 34,4 |
| acide oléique | 43,0 | 21,5 | 34,4 | 51.6 |
| hyclate de doxyxycline | 5,0 | 5,0 | 5.0 | 5.0 |
| eau | 9,0 | 9,0 | 9,0 | 9,0 |

### 1.4. Préparation au chlorhydrate de minocycline.

Les préparations ayant les compositions présentées dans le Tableau 4 sont obtenues suivant le mode opératoire général a.

**Tableau 4 -**

| Compositions G et H - Minocycline (parties) | | | | |
|---|---|---|---|---|
| Composition | G₁ | G₂ | H₁ | H₂ |
| Phosal 50 PG™ | 45,5 | 77,4 | - | - |
| NAT 8449™ | - | - | 68,3 | 45,5 |
| acide oléique | 45,5 | 13,6 | 22,7 | 45,5 |
| chlorhydrate de minocycline | 5,0 | 5,0 | 5,0 | 5,0 |
| eau | 4,0 | 4,0 | 4,0 | 4,0 |

### 1.5. Préparation à l'alcool 1,2-dichlorobenzylique

Les préparations ayant les compositions présentées dans le Tableau 5 sont obtenues suivant le mode opératoire général a.

**Tableau 5 -**

| Compositions I et J - Alcool 1,2-dichlorobenzylique (parties) | | |
|---|---|---|
| Composition | I | J |
| Phosal 50 PG™ | 80 | - |
| NAT 8449™ | - | 80 |
| acide oléique | 19 | 19 |
| alcool 1,2-dichlorobenzylique | 1 | 1 |

### 1.6. Préparation au succinate d'hydrocortisone.

Les préparations ayant les compositions présentées dans le Tableau 6 sont obtenues suivant le mode opératoire général b.

**Tableau 6 -**

| Compositions K et L - Hydrocortisone (parties) | | |
|---|---|---|
| Composition | K | L |
| Phosal 50 PG™ | 80 | - |
| NAT 8449™ | - | 67.0 |
| acide oléique | 15 | 28,0 |
| succinate d'hydrocortisone | 1 | 1 |
| eau | 4 | 4 |

### 1.7. Préparation au chlorhydrate de lidocaïne.

Les préparations ayant les compositions présentées dans le Tableau 7 sont obtenues suivant le mode opératoire général b.

**Tableau 7 -**

| Compositions M et N - Lidocaïne (parties) | | |
|---|---|---|
| Composition | M | N |
| Phosal 50 PG™ | 80 | - |
| NAT 8449™ | - | 66 |
| acide oléique | 14 | 28 |
| chlorhydrate de lidocaïne | 2 | 2 |
| eau | 4 | 4 |

### 1.8. Préparation à la somatostatine.

Les préparations ayant les compositions présentées dans le tableau 8 sont obtenues suivant les modes opératoires suivants: préparation Z₁: mode opératoire a; préparations Z₂ à Z₅: mode opératoire b.

**Tableau 8 -**

| Compositions Z₁ à Z₅ - Somatostatine (parties) | | | | | |
|---|---|---|---|---|---|
| Composition | Z₁ | Z₂ | Z₃ | Z₄ | Z₅ |
| Phosal 50 PG™ | 85 | 62,25 | 74,70 | 62,25 | 74,70 |
| PEG 400 | - | - | 8.30 | - | 8,30 |
| Propylène glycol | - | 20,75 | - | 20,75 | - |
| acide oléique | 14,95 | 13 | 13 | 13 | 13 |
| Somatostatine | 0,05 | 0,05 | 0,05 | 0,05 | 0,05 |
| tampon acétique | - | 3,95 | 3,95 | - | - |
| tampon acétique + lauryl sulfate de Na 7,5% | - | - | - | 3.95 | 3,95 |

### Exemple 2. Tests de libération.

2.1. Les préparations A₂ et B₁ préparées à l'exemple 1 ont été soumises à un test de libération effectué selon les normes de la 23 ème édition de la pharmacopée U.S. (USP 23), utilisant l'appareil n°1 à une température de 37°C, les pales tournant à 50 tpm.
   Ce test a montré que la préparation A₂ libère environ 60% du principe actif en 6 heures, la libération se poursuivant ensuite lentement pour atteindre environ 65% en 24 heures. Quant à la préparation B₁, elle libère environ 45% du principe actif en 6 heures, puis la libération continue lentement pour atteindre environ 55% en 24 heures.
2.2. Les préparations Z₁ à Z₅ préparées à l'exemple 1 ont été soumises à un test de libération effectué selon les normes de la 23 ème édition de la pharmacopée U.S. (USP 23), utilisant l'appareil n°1 à une température de 37°C, les pales tournant à 50 tpm.

Ce test a montré que la préparation Z₅ libère environ 23% du principe actif en 24 heures, la libération se poursuivant pour atteindre environ 31% en 48 heures; la préparation Z₃ libère environ 18% du principe actif en 24 heures ; la préparation Z₁ libère environ 14% du principe actif en 24 heures ; les préparations Z₂ et Z₄ libèrent environ 7% du principe actif en 24 heures. Ces résultats montrent qu'il est possible d'influencer la libération du principe actif en modifiant la composition des préparations.

### Exemple 3.

Cet exemple montre que différents solvants pharmaceutiquement acceptables peuvent être utilisés dans les compositions selon la présente invention.
3.1. Composition O : du Phospholipon 90™ (30 parties en poids) est dissous à chaud dans le polyéthylène glycol 400 (45 parties en poids). Après refroidissement, l'acide oléique est ajouté sous agitation. Au contact d'une solution aqueuse, la préparation gélifie instantanément.
   Cet exemple montre que le propylène glycol peut être remplacé par du PEG 400.
3.2. Compositions P : on mélange sous agitation 40.9 parties de NAT 8449™, 27,3 parties de PEG 400. 22,8 parties en poids d'acide oléique. De l'eau (9 parties en poids) est ajoutée sous agitation pour rendre la préparation plus visqueuse.

Les préparations ayant les compositions présentées dans le Tableau 9 sont obtenues suivant ce mode opératoire.

**Tableau 9 -**

| Compositions P (parties) | | | |
|---|---|---|---|
| Composition | P₁ | P₂ | P₃ |
| NAT 8449™ | 34,1 | 40,9 | 61,4 |
| PEG 400 | 34,1 | 27,3 | 6,8 |
| acide oléique | 22,8 | 22,8 | 22,8 |
| eau | 9,0 | 9,0 | 9,0 |

### Exemple 4.

Cet exemple montre que les compositions selon la présente invention peuvent également contenir des triglycérides.

Composition Q: On mélange sous agitation 61,2 parties de Phosal 50 PG™, 20.4 parties de Phosal 53 MCT™ et 14.4 parties d'acide oléique. On ajoute ensuite 4 parties d'eau à ce mélange sous agitation.

Cette préparation gélifie instantanément au contact d'une phase aqueuse.

### Exemple 5.

Cet exemple montre que les compositions selon la présente invention peuvent contenir différents types de phospholipides. Les phospholipides utilisés sont le sel sodique du 3-(3-sn-phosphatidyl)glycérol de soja (Phospholipon G-Na™), la 1,2-dicaproyl-sn-glycéro(3)phosphocholine (Phospholipon CC™), le sel sodique du 1,2-distéaroyl-sn-glycéro (3) phosphoglycérol (Phospholipon SG-Na™) et la (3-sn-phosphatidyl)choline de soja hydrogénée (Phospholipon 90H™)

Les compositions P présentées dans le Tableau 10 sont obtenues en mélangeant les divers composants sous agitation. Ces quatre compositions gélifient instantanément en présence d'une phase aqueuse.

**Tableau 10 -**

| Compositions R (parties) | | | | |
|---|---|---|---|---|
| Composition | R₁ | R₂ | R₃ | R₄ |
| Phospholipon G-Na™ 30 | - | - | - | |
| Phospholipon CC™ | - | 30 | - | - |
| Phospholipon SG-Na™ | - | - | 15 | - |
| Phospholipon 90H™ | - | - | - | 3 |
| PEG 400 | 45 | 45 | 60 | 72 |
| acide oléique | 25 | 25 | 25 | 25 |

### Exemple 6.

Cet exemple montre que l'acide oléique peut être remplacé par d'autres acides gras ou par un alcool gras dans les compositions selon la présente invention.

Les compositions S présentées dans le Tableau 11 sont obtenues en mélangeant les divers composants sous agitation. Ces quatre compositions gélifient instantanément en présence d'une phase aqueuse.

**Tableau 11 -**

| Compositions S (parties) | | | | |
|---|---|---|---|---|
| Composition | S₁ | S₂ | S₃ | S₄ |
| Phosal 50PG™ | 80 | 80 | 80 | 80 |
| acide caprylique | 20 | - | - | - |
| acide caprique | - | 20 | - | - |
| acide oléique | - | - | 20 | - |
| alcool oléique | - | - | - | 20 |

### Exemple 7. Mesure de la vitesse de libération en fonction des excipients.

7.1. Les compositions T présentées dans le Tableau 12 sont obtenues en ajoutant la quantité voulue d'une solution aqueuse à 10% de colorant Sicomet-FDC bleu 1 au mélange des autres composants sous agitation.
Les compositions T₁ à T₆ gélifient instantanément en présence d'une phase aqueuse: le gel est plus fluide pour la composition T₇.

**Tableau 12 -**

| Compositions T (parties) | | | | | | | | |
|---|---|---|---|---|---|---|---|---|
| Composition | contrôle | T₁ | T₂ | T₃ | T₄ | T₅ | T₆ | T₇ |
| Phosal 50PG™ | - | 81,6 | 68,3 | 68,3 | 68,3 | 68,3 | 68,3 | 40,8 |
| acide oléique | - | 14,4 | 22,7 | 18,2 | 13,7 | 18,2 | 9,0 | 14,4 |
| Miglyol 810N™ | - | - | - | 4,5 | 9,0 | - | 13,7 | - |
| Phosal 53 MCT™ | - | - | - | - | - | 4,5 | - | 40,8 |
| solution de colorant | 100 | 4,0 | 9,0 | 9,0 | 9,0 | 9,0 | 9,0 | 4,0 |

Le test de libération est effectué comme suit. Des quantités égales des préparations T₁ à T₇ et de la solution de contrôle sont déposées dans un puits creusé au centre d'une couche d'épaisseur constante de trypticase soy agar coulée dans une boîte de Pétri. La vitesse de diffusion du colorant est déterminée en mesurant le diamètre de la tache de colorant en fonction du temps. Les résultats obtenus pour la solution de contrôle et les solutions T₁ à T₇ sont repris dans le Tableau 13.

**Tableau 13 -**

| Vitesse de libération des préparations T₁ à T₇. | | | | | | | |
|---|---|---|---|---|---|---|---|
| Temps (heures) | Diamètre de la tache en mm. | | | | | | |
| | contrôle | T₁ | T₂ | T₃ | T₄ | T₅ | T₆, T₇ |
| 0 | 16,97 | 18,74 | 18,61 | 17,66 | 18,36 | 18,49 | * |
| 3 | 49,55 | - | - | - | - | - | * |
| 6 | 62,18 | 29,56 | 29,14 | 28,58 | 23,12 | 33,33 | * |
| 24 | 90,10 | 51,00 | 30,38 | 30,57 | 24,59 | 52,96 | * |
| 36 | 96,29 | 57,45 | 34,02 | 33,67 | 31,23 | 54,55 | * |
| 72 | 108,52 | 60,45 | 39,29 | 34,58 | 31,82 | 68,67 | * |

| | | | | | | | |
|---|---|---|---|---|---|---|---|
| * Aucune diffusion n'est observée. | | | | | | | |

Cet exemple montre que l'on peut contrôler la vitesse de libération d'une substance active par le choix des composants de la préparation.
7.2. De manière analogue, on a préparé les compositions U reprises dans le Tableau 14.

**Tableau 14 -**

| Compositions U (parties) | | | | |
|---|---|---|---|---|
| Composition | U ₁ | U₂ | U₃ | U₄ |
| Phosal 50PG™ | 72,0 | 81,6 | 86,4 | 91,2 |
| acide oléique | 24,0 | 14,4 | 9,6 | 4,8 |
| solution de colorant | 4,0 | 4,0 | 4,0 | 4,0 |

Un test de libération tel que décrit à l'exemple 7.1 est effectué sur les compositions U1 à U4; pour comparaison, un test de libération est simultanément réalisé avec la solution T7 et avec une solution à 10% de Sicomet-FDC blue 1 (contrôle). Les résultats de ce test sont présentés au Tableau 15.

**Tableau 15 -**

| Vitesse de libération des préparations U ₁ à U₄ et T₇. | | | | | | |
|---|---|---|---|---|---|---|
| Temps (heures) | Diamètre de la tache en mm. | | | | | |
| | contrôle | U ₁ | U₂ | U₃ | U₄ | T₇ |
| 2 | 46 | 18 | 24 | 27 | 24 | 18 |
| 4 | 55 | 18 | 28 | 34 | 26 | 18 |
| 6 | 62 | 18 | 33 | 40 | 28 | 18 |
| 24 | 82 | 23 | 48 | 47 | 37 | 18 |

Ces résultats montrent que l'on peut contrôler la vitesse de libération d'une substance active par le choix des composants de la préparation.

### Exemple 8. Essais in vivo. Injection sous-cutanée et intramusculaire d'une préparation à la calcitonine.

La calcitonine provoque une diminution du taux de calcium sérique en relation directe avec son activité. Au cours de ces essais, l'évolution au cours du temps du taux de calcium sérique chez le rat a été suivie après injection sous-cutanée ou intramusculaire de préparations selon l'invention contenant 20 UI de calcitonine de saumon.

### 8.1. Formulations.

Les compositions comprenant de la calcitonine de saumon utilisées dans ces essais sont reprises dans le Tableau 16.

**Tableau 16 -**

| Compositions X (parties) | | | |
|---|---|---|---|
| Composition | X₁ | X₂ | X₃ |
| Phosal 50PG™ | - | 40,8 | 81,6 |
| Phosal 53 MCT™ | - | 40,8 | - |
| acide oléique | - | 14,4 | 14,4 |
| calcitonine | 10000 UI | 10000 UI | 10000 UI |
| tampon acétique pH 4,3 | 100 | 4,0 | 4,0 |

La calcitonine utilisée contient 5660 UI/mg. Les 10000 UI présentes dans les préparations X₁ à X₃ correspondent à 1,767 mg.

### 8.2. Expérimentation animale.

L'expérimentation a porté sur deux groupes de 18 rats Wistar mâles conscients et non mis à jeun (provenance IFFA CREDO) d'un poids de 169,1 g à 193,6 g (moyenne : 183,0 g ; écart-type : 5,9 g), pour le premier groupe, et d'un poids de 170,2 g à 189,1 g (moyenne : 180,0 g : écart-type : 5,2 g), pour le second groupe. Chaque groupe de 18 animaux est réparti en 3 séries de 6 :
Premier groupe:
   - série 1 : chaque rat reçoit par voie sous-cutanée dans la région abdominale 200 µl de la préparation X₁, soit 20 UI de calcitonine;
   - série 2 : chaque rat reçoit par voie sous-cutanée dans la région abdominale 200 µl de la préparation X₂, soit 20 UI de calcitonine;
   - série 3 : chaque rat reçoit par voie sous-cutanée dans la région abdominale 200 µl de la préparation X₂, soit 20 UI de calcitonine.
Second groupe:
   - série 4 : chaque rat reçoit par voie intramusculaire dans le muscle de la cuisse 200 µl de la préparation X₁, soit 20 UI de calcitonine;
   - série 5 : chaque rat reçoit par voie intramusculaire dans le muscle de la cuisse 200 µl de la préparation X₂, soit 20 UI de calcitonine;
   - série 6 : chaque rat reçoit par voie intramusculaire dans le muscle de la cuisse 200 µl de la préparation X₂, soit 20 UI de calcitonine.

Après administration des préparations, les rats reçoivent une nourriture pauvre en calcium et de l'eau désionisée.

Des échantillons sanguins de 300 µL sont prélevés au niveau d'une veine caudale avant administration (t = 0) et aux temps suivants après administration: 30 min, 1 h, 2 h, 4 h, 8 h, 24 h, 32 h et 48 h. Les échantillons reposent 1 h à température ambiante avant de subir 2 centrifugations successives à 6.000 tpm durant 10 minutes. Les sera recueillis sont congelés à -20°C jusqu'au moment du dosage du calcium sérique.

### 8.3. Dosage du calcium sérique.

Un échantillon sérique de 90 µL est ajouté à 2 mL une solution de chlorure de lanthane (15 mmol/L) dans l'acide chlorhydrique (50 mmol/L). Le taux calcique de l'échantillon ainsi dilué est mesuré à l'aide d'un spectrophotomètre d'absorption atomique (Varian Spectra A-40) (Extinction à 422,7 nm). La courbe d'étalonnage est réalisée au départ de 5 solutions standard :
- un blanc contenant du chlorure de sodium (140 mmol/L), du chlorure de potassium (5 mmol/L), de l'acide chlorhydrique (30 mmol/L) et de l'acétate de magnésium (1 mmol/L)
- les solutions standard contenant en plus du blanc du carbonate de calcium aux concentrations de 1,25/2,5/5 et 7,5 mmol/L.

L'appareil est étalonné avant chaque série de rats. Le taux calcique des échantillons est calculé à partir de la courbe d'étalonnage (Data Station Varian). Les concentrations calculées sont ensuite exprimées en pourcentage de la valeur initiale c'est-à-dire la valeur obtenue avant tout traitement (t=0). Ces valeurs initiales varient selon l'animal de 3,34 à 2,26 mmol/L.

Les résultats obtenus avec les séries 1, 2 et 3 (administration sous-cutanée) sont présentés au Tableau 17 qui reprend les moyennes des taux sériques de calcium exprimés en pourcentage de la concentration initiale (t=0) et les erreurs type sur ces moyennes obtenus pour les préparations X₁, X₂ et X₃.

**Tableau 17 -**

| Taux sériques de calcium ± erreur type en fonction du temps après injection sous-cutanée des formulations X₁, X₂ et X₃. | | | |
|---|---|---|---|
| Temps (h) | X₁ | X₂ | X₃ |
| 0 | 100 | 100 | 100 |
| 0,5 | 86,52 ± 2,90 | 91,11 ± 1.59 | 90,72 ± 2,17 |
| 1 | 80,33 ± 4,29 | 84,00 ± 2,05 | 86,59 ± 2,39 |
| 2 | 69,13 ± 1,79 | 74,43 ± 2,38 | 75,69 ± 1,62 |
| 4 | 59,26 ± 0,86 | 73,62 ± 4,41 | 69,19 ± 2,39 |
| 8 | 55,04 ± 1,12 | 82,30 ± 7,26 | 66.52 ± 1,13 |
| 24 | 97,14 ± 2,38 | 95,65 ± 3,93 | 73,38 ± 3,59 |
| 32 | 101,03 ± 2,99 | 93,85 ± 3,90 | 75,98 ± 2,40 |
| 48 | 97,32 ± 3,44 | 100,09 ± 1,75 | 87,13 ± 1.51 |

Les résultats obtenus avec les séries 4, 5 et 6 (administration intramusculaire) sont présentés au Tableau 18 qui reprend les moyennes des taux sériques de calcium exprimés en pourcentage de la concentration initiale (t=0) et les erreurs type sur ces moyennes obtenus pour les préparations X₁, X₂ et X₃.

**Tableau 18 -**

| Taux sériques de calcium ± erreur type en fonction du temps après injection intramusculaire des formulations X₁, X₂ et X₃. | | | |
|---|---|---|---|
| Temps (h) | X₁ | X₂ | X₃ |
| 0 | 100 | 100 | 100 |
| 0,5 | 86,51 ± 2,09 | 89,06 ± 1,39 | 88,27 ± 1,64 |
| 2 | 78,77 ± 1,80 | 79,31 ± 1.55 | 81,81 ± 1.86 |
| 2 | 73,89 ± 1,82 | 75,82 ± 2,52 | 75,95 ± 1,31 |
| 4 | 65,63 ± 1,32 | 68,58 ± 2,14 | 72,74 ± 1,57 |
| - 8 | 62,93 ± 1,55 | 63,91 ± 1,45 | 66,01 ± 1,55 |
| 24 | 83,70 ± 3,25 | 78,66 ± 4,07 | 74,12 ± 2,38 |
| 32 | 94,50 ± 1,70 | 85,83 ± 4,41 | 78,12 ± 2,24 |
| 48 | 98.53 ± 2,76 | 105,33 ± 4,15 | 100,62 ± 1,34 |

Ces résultats montrent une prolongation de l'effet de la calcitonine, après administration sous-cutanée ou intramusculaire, de plusieurs heures pour les préparations X₂ et X₃ par rapport à l'effet de la solution de référence X₁, cet effet étant plus important pour la préparation X₃ que pour la préparation X₂.

Ces essais montrent aussi qu'il est possible de moduler in vivo l'activité biologique du principe actif en modifiant la composition des préparations. Ceci apparaît clairement dans le tableau 19 qui reprend les biodisponibilités relatives (par rapport à la solution X₁) des préparations X₂ et X₃ après injections sous-cutanée (s.c.) et intramusculaire (i.m.)

**Tableau 19 -**

| Biodisponibilité relative (%) des préparations X₂ et X₃. | | |
|---|---|---|
| Injection | X₂ | X₃ |
| sous-cutanée | 63,89 | 172,56 |
| intramusculaire | 110,41 | 128,72 |

## Revendications

1. Composition pharmaceutique fluide permettant la libération contrôlée d'au moins une substance active comprenant
a) une quantité thérapeutiquement efficace d'au moins une substance active.
b) de 3 à 55% en poids de phospholipide,
c) de 16 à 72% en poids de solvant pharmaceutiquement acceptable, et
d) de 4 à 52% en poids d'acide gras,
ladite composition ayant la propriété de se gélifier instantanément en présence d'une phase aqueuse.

2. Composition pharmaceutique selon la revendication 1, **caractérisée en ce que** la substance active est choisie parmi les antibiotiques, les agents anti-infectieux, les anesthésiques locaux, les anti-inflammatoires, les antimycosiques ou des substances actives peptidiques.

3. Composition pharmaceutique selon la revendication 1 ou 2, **caractérisée en ce que** le phospholipide est choisi parmi la phosphatidylcholine, les sels du phosphatidylglycérol, la dicaproylphosphatidylcholine et les sels du distéaroylphosphatidylglycérol, seuls ou en mélange.

4. Composition pharmaceutique selon la revendication 3, **caractérisée en ce qu'**elle contient de 15 à 55% en poids de phospholipide, de préférence, de 15 à 51% en poids de phospholipide.

5. Composition pharmaceutique selon la revendication 1 ou 2, **caractérisée en ce que** le phospholipide est une phosphatidylcholine hydrogénée.

6. Composition selon la revendication 5, **caractérisée en ce qu'**elle contient de 3 à 11%, de préférence de 3 à 10% en poids de phospholipide.

7. Composition selon l'une quelconque des revendications 1 à 6, **caractérisée en ce que** le solvant pharmaceutiquement acceptable est choisi parmi le propylène glycol, les polyéthylène glycols ou les huiles minérales telles que l'huile de paraffine ou les huiles de silicone, seuls ou en mélange.

8. Composition selon l'une quelconque des revendications 1 à 7, **caractérisée en ce que** les acides gras utilisés sont des acides carboxyliques organiques saturés ou insaturés contenant de 4 à 22 atomes de carbone, de préférence de 8 à 18 atomes de carbone.

9. Composition pharmaceutique selon la revendication 8, **caractérisée en ce que** les acides gras sont choisis parmi l'acide oléique, l'acide caprylique, l'acide caprique, l'acide caproique, l'acide myristique ou l'acide butyrique, seuls ou en mélange.

10. Composition pharmaceutique selon l'une quelconque des revendications 1 à 9, **caractérisée en ce qu'**elle comprend en outre jusqu'à 5% en poids de monoglycéride ou de diglycéride ou d'un mélange de mono- et de diglycéride et/ou jusqu'à 15% en poids de triglycérides.

11. Procédé de fabrication d'une composition pharmaceutique selon l'une quelconque des revendications 1 à 10, **caractérisé en ce qu'**il comprend les étapes successives suivantes:
i) le ou les phospholipides sont dissous dans le solvant pharmaceutiquement acceptable;
ii) le ou les acides gras sont ajoutés à la solution de phospholipide sous agitation;
iii) la ou les substances actives sont incorporées au mélange obtenu à la fin de l'étape ii), et
iv) de l'eau est éventuellement ajoutée à la composition obtenue à l'étape iii),

12. Procédé selon la revendication 11, **caractérisé en ce que** la ou les substances actives sont dissoutes dans une quantité minimale d'eau avant l'incorporation à l'étape iii).

13. Procédé selon la revendication 11, **caractérisé en ce que** la ou les substances actives sont incorporées à l'étape iii), éventuellement sous forme micronisée.

14. Utilisation d'une composition selon l'une quelconque des revendications 1 à 13 pour la préparation d'une composition pour la libération contrôlée d'une ou plusieurs substances actives par injection sous-cutanée et/ou intramusculaire de substances actives.

## Patentansprüche

1. Flüssige pharmazeutische Zusammensetzung, die die kontrollierte Freisetzung wenigstens eines Wirkstoffs ermöglicht, die umfasst
a) eine therapeutisch wirksame Menge wenigstens eines Wirkstoffs,
b) 3 bis 55 Gew.-% Phospholipid,
c) 16 bis 72 Gew.-% pharmazeutisch annehmbares Lösungsmittel und
d) 4 bis 52 Gew.-% Fettsäure,
wobei besagte Zusammensetzung die Eigenschaft hat, in Gegenwart einer wässrigen Phase sofort zu gelieren.

2. Pharmazeutische Zusammensetzung gemäß Anspruch 1, **dadurch gekennzeichnet, dass** der Wirkstoff ausgewählt ist unter den Antibiotika, den Mitteln gegen Infektionen, den Lokalanästhetika, den Entzündungshemmern, den Mitteln gegen Pilze oder Peptid-Wirkstoffen.

3. Pharmazeutische Zusammensetzung gemäß Anspruch 1 oder 2, **dadurch gekennzeichnet, dass** das Phospholipid ausgewählt ist unter Phosphatidylcholin, den Salzen von Phosphatidylglycerol, Dicaproylphosphatidylcholin und den Salzen von Distearoylphosphatidylglycerol, allein oder im Gemisch.

4. Pharmazeutische Zusammensetzung gemäß Anspruch 3, **dadurch gekennzeichnet, dass** sie 15 bis 55 Gew.-% Phospholipid, vorzugsweise 15 bis 51 Gew.-% Phospholipid enthält.

5. Pharmazeutische Zusammensetzung gemäß Anspruch 1 oder 2, **dadurch gekennzeichnet, dass** das Phospholipid ein hydriertes Phosphatidylcholin ist.

6. Zusammensetzung gemäß Anspruch 5, **dadurch gekennzeichnet, dass** sie 3 bis 11 Gew.-%, vorzugsweise 3 bis 10 Gew.-% Phospholipid enthält.

7. Zusammensetzung gemäß einem der Ansprüche 1 bis 6, **dadurch gekennzeichnet, dass** das pharmazeutisch annehmbare Lösungsmittel ausgewählt ist unter Propylenglykol, den Polyethylenglykolen oder den Mineralölen, wie Paraffinöl, oder den Siliconölen, allein oder im Gemisch.

8. Zusammensetzung gemäß einem der Ansprüche 1 bis 7, **dadurch gekennzeichnet, dass** die verwendeten Fettsäuren gesättigte oder ungesättigte organische Carbonsäuren mit 4 bis 22 Kohlenstoffatomen, vorzugsweise 8 bis 18 Kohlenstoffatomen sind.

9. Pharmazeutische Zusammensetzung gemäß Anspruch 8, **dadurch gekennzeichnet, dass** die Fettsäuren ausgewählt sind unter Oleinsäure, Caprylsäure, Caprinsäure, Capronsäure, Myristinsäure oder Buttersäure, allein oder im Gemisch.

10. Pharmazeutische Zusammensetzung gemäß einem der Ansprüche 1 bis 9, **dadurch gekennzeichnet, dass** sie außerdem bis zu 5 Gew.-% Monoglycerid oder Diglycerid oder eines Gemischs von Mono- und Diglycerid und/oder bis zu 15 Gew.% Triglyceride umfasst.

11. Verfahren zur Herstellung einer pharmazeutischen Zusammensetzung gemäß einem der Ansprüche 1 bis 10, **dadurch gekennzeichnet, dass** es die folgenden aufeinander folgenden Schritte umfasst:
i) das oder die Phospholipide werden in dem pharmazeutisch annehmbaren Lösungsmittel gelöst;
ii) die Fettsäure(n) werden zu der Phospholipidlösung unter Rühren hinzugefügt;
iii) der oder die Wirkstoffe werden dem am Ende des Schritts (ii) erhaltenen Gemisch zugesetzt und
iv) Wasser wird gegebenenfalls zu der im Schritt (iii) erhaltenen Zusammensetzung hinzugefügt.

12. Verfahren gemäß Anspruch 11, **dadurch gekennzeichnet, dass** der oder die Wirkstoffe in einer minimalen Menge an Wasser vor dem Zusetzen im Schritt iii) gelöst werden.

13. Verfahren gemäß Anspruch 11, **dadurch gekennzeichnet, dass** der oder die Wirkstoffe im Schritt iii) gegebenenfalls in mikronisierter Form zugesetzt werden.

14. Verwendung einer Zusammensetzung gemäß einem der Ansprüche 1 bis 13 für die Herstellung einer Zusammensetzung für die kontrollierte Freisetzung eines oder mehrerer Wirkstoffe durch subkutane und/oder intramuskuläre Injektion von Wirkstoffen.

## Claims

1. Fluid pharmaceutical composition for the controlled release of at least one active substance comprising:
a) a therapeutically effective quantity of at least one active substance,
b) from 3 to 55% by weight of phospholipid,
c) from 16 to 72% by weight of a pharmaceutically acceptable solvent, and
d) from 4 to 52% by weight of fatty acid,
the said composition having the property of gelling instantaneously in the presence of an aqueous phase.

2. Pharmaceutical composition according to claim 1, **characterized in that** the active substance is chosen from antibiotics, anti-infective agents, local anaesthetics, anti-inflammatories, antimycotics or peptidic active substances.

3. Pharmaceutical composition according to claim 1 or 2, **characterized in that** the phospholipid is chosen from phosphatidylcholine, phosphatidylglycerol salts, dicaproyl phosphatidylcholine and distearoylphosphatidylglycerol salts, alone or mixed.

4. Pharmaceutical composition according to claim 3, **characterized in that** it contains 15 to 55% by weight of phospholipid, preferably 15 to 51% by weight of phospholipid.

5. Pharmaceutical composition according to claim 1 or 2, **characterized in that** the phospholipid is a hydrogenated phosphatidylcholine.

6. Pharmaceutical composition according to claim 5, **characterized in that** it contains 3 to 11%, preferably 3 to 10% by weight of phospholipid.

7. Pharmaceutical composition according to any one of claims 1 or 6, **characterized in that** the pharmaceutically acceptable solvent is chosen from propylene glycol, polyethylene glycols or mineral oils such as paraffin oil or silicone oils, alone or mixed.

8. Pharmaceutical composition according to any one of claims 1 to 7, **characterized in that** the fatty acids used are saturated or unsaturated organic carboxylic acids containing 4 to 22 carbon atoms, preferably 8 to 18 carbon atoms.

9. Composition according to claim 8, **characterized in that** the fatty acids are chosen from oleic acid, caprylic acid, capric acid, caproic acid, myristic acid or butyric acid, alone or mixed.

10. Pharmaceutical composition according to any one of claims 1 to 9, **characterized in that** it additionally includes up to 5% by weight of mono- or diglyceride or a mixture of mono- and diglyceride and/or up to 15% by weight of triglycerides.

11. Method for producing a pharmaceutical composition according to any one of claims 1 to 10, **characterized in that** it comprises the following successive stages:
i) the phospholipid(s) is/are dissolved in the pharmaceutically acceptable solvent;
ii) the fatty acid(s) is/are added to the phospholipid solution with stirring;
iii) the active substance(s) is/are incorporated in the mixture obtained at the end of step ii), and
iv) water is optionally added to the composition obtained in step iii).

12. Method according to claim 11, **characterized in that** the active substance(s) is/are dissolved in a minimum quantity of water before being incorporated in step iii).

13. Method according to claim 11, **characterized in that** the active substance(s) is/are incorporated in step iii), optionally in micronised form.

14. Use of a composition according to any one of claims 1 to 13 for the preparation of a composition for the controlled release of one or more active substances by subcutaneous and/or intramuscular injection of the active substances.
